# EUROPEAN PATENT APPLICATION

(11) **EP 1 925 621 A1**
(43) Date of publication of application: **28.05.2008**
(21) Application number: 06124850.6
(22) Date of filing: 27.11.2006
(51) Int. Cl.: C07F 9/6506, A61K 31/675, A61P 35/00

(54) **Crystalline forms of zoledronic acid**

(71) Applicant: Novartis AG, 4002 Basel (CH)
(72) Inventor: The designation of the inventor has not yet been filed
(74) Representative: Roth, Peter Richard

(57) **Abstract**

The invention relates to new crystalline forms of low water soluble salts of zoledronic acid, the process for preparation of these crystalline forms, compositions containing these crystalline forms, and the use of these crystalline forms in diagnostic methods or therapeutic treatment of warm-blooded animals, especially humans. The invention relates to the crystalline form of the free acid monohydrate of zoledronic acid, the process for preparation of the crystalline form of the free acid monohydrate of zoledronic acid, compositions containing the crystalline form of the free acid monohydrate of zoledronic acid, and the use of crystalline form of the free acid monohydrate of zoledronic acid in diagnostic methods or therapeutic treatment of warm-blooded animals, especially humans.

## Description

The invention relates to new crystalline forms of low water soluble salts of zoledronic acid, the process for preparation of these crystalline forms, compositions containing these crystalline forms, and the use of these crystalline forms in diagnostic methods or therapeutic treatment of warm-blooded animals, especially humans.

The invention relates to the crystalline monohydrate of the free acid of zoledronic acid, the process for preparation of the crystalline monohydrate of the free acid of zoledronic acid, compositions containing the crystalline monohydrate of the free acid of zoledronic acid, and the use of crystalline monohydrate of the free acid of zoledronic acid in diagnostic methods or therapeutic treatment of warm-blooded animals, especially humans.

The invention relates to the crystalline trihydrate of the free acid of zoledronic acid, the process for preparation of the crystalline trihydrate of the free acid of zoledronic acid, compositions containing the crystalline trihydrate of the free acid of zoledronic acid, and the use of crystalline trihydrate of the free acid of zoledronic acid in diagnostic methods or therapeutic treatment of warm-blooded animals, especially humans.

The invention relates to the crystalline anhydrous form of the free acid of zoledronic acid, the process for preparation of the crystalline anhydrous form of the free acid of zoledronic acid, compositions containing the crystalline anhydrous form of the free acid of zoledronic acid, and the use of crystalline anhydrous form of the free acid of zoledronic acid in diagnostic methods or therapeutic treatment of warm-blooded animals, especially humans.

The invention also relates to the amorphous form of the free acid of zoledronic acid, the process for preparation of the amorphous form of the free acid of zoledronic acid, compositions containing the amorphous form of the free acid of zoledronic acid, and the use of the amorphous form of the free acid of zoledronic acid in diagnostic methods or therapeutic treatment of warm-blooded animals, especially humans.

### Background of the Invention

The drug zoledronic acid is used in the prevention of skeletal related events, (pathological fractures, spinal compression, radiation or surgery to bone, or tumor-induced hypercalcemia) in patients with advanced malignancies involving bone; treatment of tumor-induced hypercalcemia; Paget's disease, OP and prevention of recurrent hip fractures. In general, the preparation of zoledronic acid is known in the art. However, it is also known that different polymorphic forms of the same drug may have substantial differences in certain pharmaceutically important properties. Therefore, there is a continuing need for new solid forms of zoledronic acid and new methods of preparation.

### Summary of the Invention

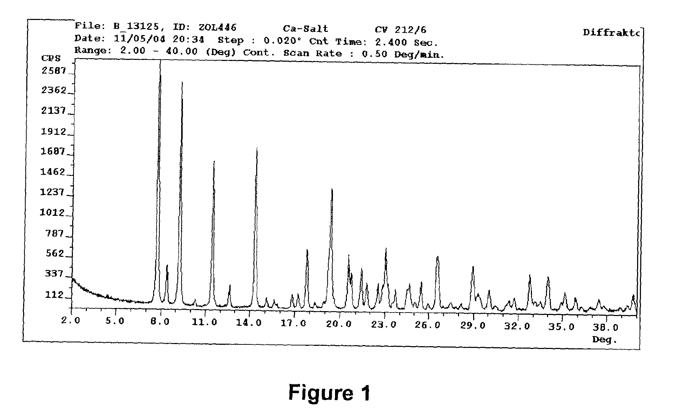

In accordance with one aspect, the invention provides a crystalline form of the calcium salt of zoledronic acid with a stoichiometry of one calcium and two zoledronic acid molecules, also known as "1:2 calcium salt of zoledronic acid". Preferably, the crystalline form of the calcium salt of zoledronic acid with a stoichiometry of one calcium and two zoledronic acid molecules has an X-ray diffraction pattern with a peak at an angle of refraction 2 theta (θ) of 7.8, 8.4, 9.3, 11.5, 14.3, 17.8, 19.4, 23.1 ± 0.2 as depicted in Figure 1.

In accordance with yet another aspect, the invention provides a composition that contains zoledronic acid in a solid form, wherein at least 80% by weight of the solid zoledronic acid is its crystalline form of the calcium salt of zoledronic acid with a stoichiometry of one calcium and two zoledronic acid molecules having an X-ray diffraction pattern with a peak at an angle of refraction 2 θ of 7.8, 8.4, 9.3, 11.5, 14.3, 17.8, 19.4, 23.1 ± 0.2 as depicted in Figure 1. Various embodiments and variants are provided.

In accordance with yet another aspect, the invention provides a pharmaceutical composition that includes crystalline form of the calcium salt of zoledronic acid with a stoichiometry of one calcium and two zoledronic acid molecules and a pharmaceutically acceptable carrier or diluent. Preferably, the pharmaceutical composition is for oral administration.

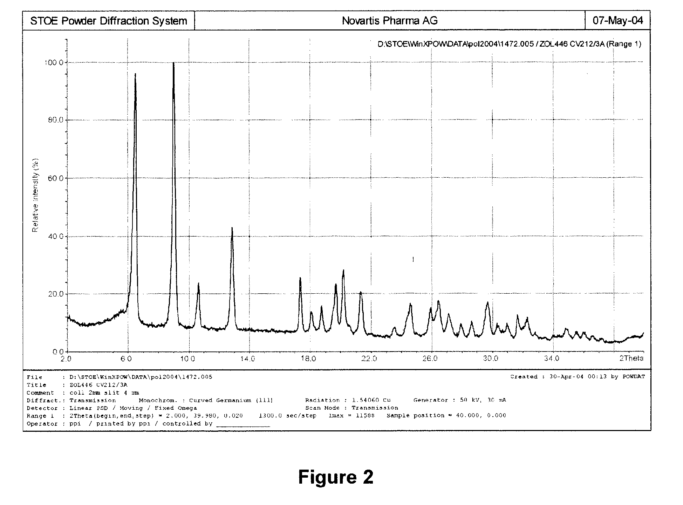

In accordance with one aspect, the invention provides a crystalline form of the calcium salt of zoledronic acid with a stoichiometry of one calcium and one zoledronic acid molecule, also known as "1:1 calcium salt of zoledronic acid". Preferably, the crystalline form of the calcium salt of zoledronic acid has an X-ray diffraction pattern with a peak at an angle of refraction 2 θ of 5.7, 6.5, 9.0, 10.6, 12.9, 17.4, 18.1, 18.8, 19.7, 20.2 ± 0.2 deg as depicted in Figure 2.

In accordance with yet another aspect, the invention provides a composition that contains zoledronic acid in a solid form, wherein at least 80% by weight of the solid zoledronic acid is its crystalline form of the calcium salt of zoledronic acid with a stoichiometry of one calcium and one zoledronic acid molecule having an X-ray diffraction pattern with a peak at an angle of refraction 2 θ of 5.7 and 6.5 ± 0.2 as depicted in Figure 2. Various embodiments and variants are provided.

In accordance with yet another aspect, the invention provides a pharmaceutical composition that includes crystalline form of the calcium salt of zoledronic acid with a stoichiometry of one calcium and one zoledronic acid molecule and a pharmaceutically acceptable carrier or diluent. Preferably, the pharmaceutical composition is for oral administration.

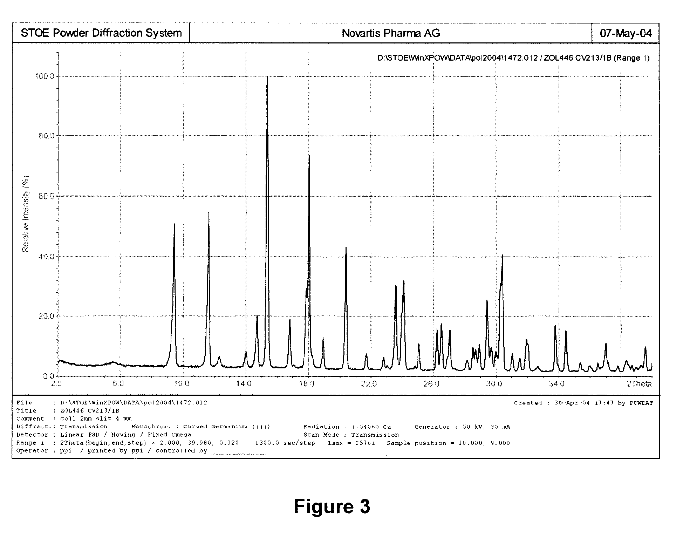

In accordance with one aspect, the invention provides a crystalline form I of the zinc salt of zoledronic acid with a stoichiometry of one zinc and two zoledronic acid molecules. Preferably, the crystalline form I of the zinc salt of zoledronic acid with a stoichiometry of one zinc and two zoledronic acid molecules has an X-ray diffraction pattern with a peak at an angle of refraction 2 θ of 9.2, 9.5, 11.7, 15.5,18.1, 20.5, 23.7, 24.3 ± 0.2 deg as depicted in Figure 3.

In accordance with yet another aspect, the invention provides a composition that contains zoledronic acid in a solid form, wherein at least 80% by weight of the solid zoledronic acid is its crystalline form I of the zinc salt of zoledronic acid with a stoichiometry of one zinc and two zoledronic acid molecules having an X-ray diffraction pattern with a peak at an angle of refraction 2 θ of 9.2, 9.5, 11.7, 15.5,18.1, 20.5, 23.7, 24.3 ± 0.2 as depicted in Figure 3. Various embodiments and variants are provided.

In accordance with yet another aspect, the invention provides a pharmaceutical composition that includes crystalline form I of the zinc salt of zoledronic acid with a stoichiometry of one zinc and two zoledronic acid molecules and a pharmaceutically acceptable carrier or diluent. Preferably, the pharmaceutical composition is for oral administration.

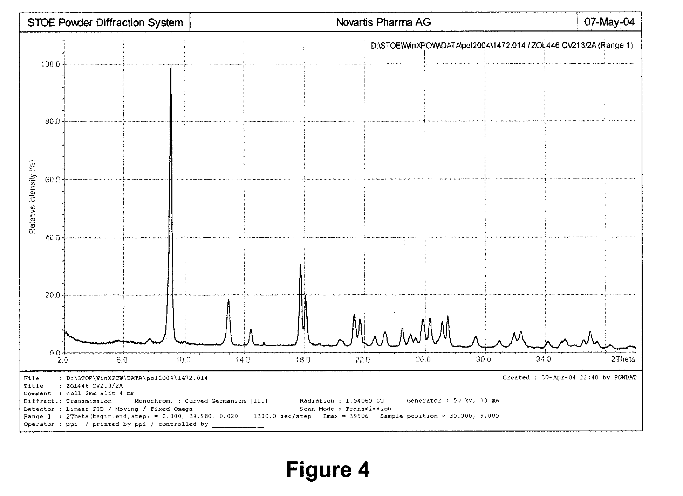

In accordance with one aspect, the invention provides a crystalline form II of the zinc salt of zoledronic acid with a stoichiometry of one zinc and two zoledronic acid molecules. Preferably, the crystalline form II of the zinc salt of zoledronic acid with a stoichiometry of one zinc and two zoledronic acid molecules has an X-ray diffraction pattern with a peak at an angle of refraction 2 θ of 9.1, 13.0, 17.7, 18.0 ± 0.2 as depicted in Figure 4.

In accordance with yet another aspect, the invention provides a composition that contains zoledronic acid in a solid form, wherein at least 80% by weight of the solid zoledronic acid is its crystalline form II of the zinc salt of zoledronic acid with a stoichiometry of one zinc and two zoledronic acid molecules having an X-ray diffraction pattern with a peak at an angle of refraction 2 θ of 9.1 ± 0.2 as depicted in Figure 4. Various embodiments and variants are provided.

In accordance with yet another aspect, the invention provides a pharmaceutical composition that includes crystalline form II of the zinc salt of zoledronic acid with a stoichiometry of one zinc and two zoledronic acid molecules and a pharmaceutically acceptable carrier or diluent. Preferably, the pharmaceutical composition is for oral administration.

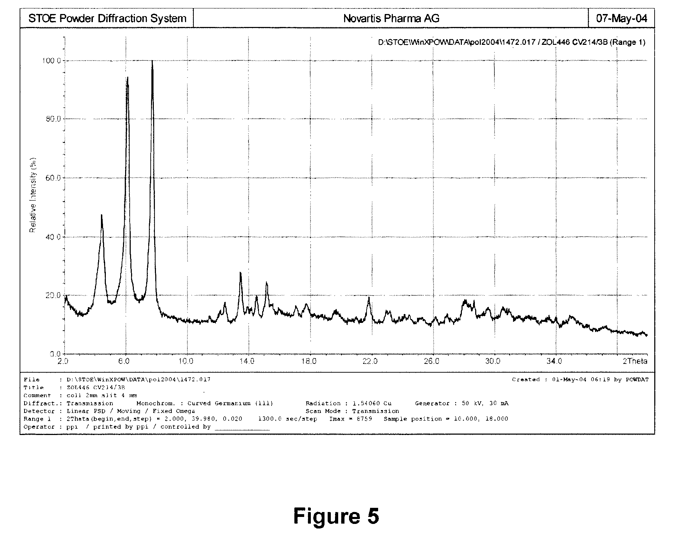

In accordance with one aspect, the invention provides a crystalline form of the magnesium salt of zoledronic acid with a stoichiometry of one magnesium and two zoledronic acid molecules, also known as "1:2 magnesium salt of zoledronic acid". Preferably, the crystalline form of the magnesium salt of zoledronic acid with a stoichiometry of one magnesium and two zoledronic acid molecules has an X-ray diffraction pattern with a peak at an angle of refraction 2 θ of 4.5, 6.1, 7.7 ± 0.2 as depicted in Figure 5.

In accordance with yet another aspect, the invention provides a composition that contains zoledronic acid in a solid form, wherein at least 80% by weight of the solid zoledronic acid is its crystalline form of the magnesium salt of zoledronic acid with a stoichiometry of one magnesium and two zoledronic acid molecules having an X-ray diffraction pattern with a peak at an angle of refraction 2 θ of 6.1, 7.7 ± 0.2 as depicted in Figure 5. Various embodiments and variants are provided.

In accordance with yet another aspect, the invention provides a pharmaceutical composition that includes crystalline form of the magnesium salt of zoledronic acid with a stoichiometry of one magnesium and two zoledronic acid molecules and a pharmaceutically acceptable carrier or diluent. Preferably, the pharmaceutical composition is for oral administration.

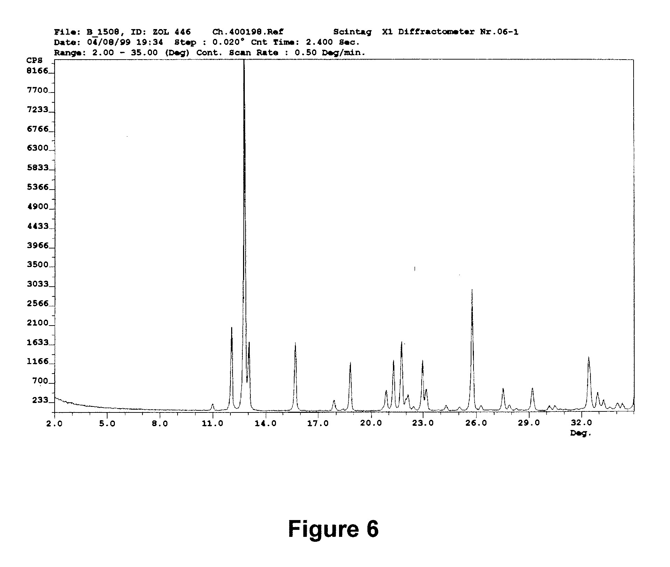

In accordance with one aspect, the invention provides a crystalline monohydrate of the free acid of zoledronic acid. Preferably, the crystalline monohydrate of the free acid of zoledronic acid has an X-ray diffraction pattern with a peak at an angle of refraction 2 θ of 12.0, 12.8, 15.7, 18.8, 21.2, 21.7, 22.9 ± 0.2 as depicted in Figure 6.

In accordance with yet another aspect, the invention provides a composition that contains zoledronic acid in a solid form, wherein at least 80% by weight of the solid zoledronic acid is its crystalline monohydrate of the free acid of zoledronic acid, having an X-ray diffraction pattern with a peak at an angle of refraction 2 θ of 12.8 ± 0.2 as depicted in Figure 6. Various embodiments and variants are provided.

In accordance with yet another aspect, the invention provides a pharmaceutical composition that includes crystalline monohydrate of the free acid of zoledronic acid, and a pharmaceutically acceptable carrier or diluent. Preferably, the pharmaceutical composition is for oral administration.

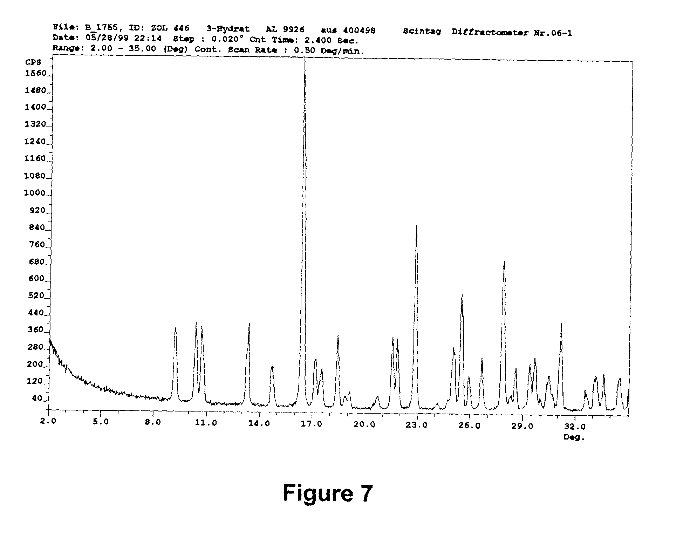

In accordance with one aspect, the invention provides a crystalline trihydrate of the free acid of zoledronic acid. Preferably, the crystalline trihydrate of the free acid of zoledronic acid has an X-ray diffraction pattern with a peak at an angle of refraction 2 θ of 9.2, 10.3, 10.7, 13.3, 16.3, 18.4, 21.5, 21.8, 22.8 ± 0.2 as depicted in Figure 7.

In accordance with yet another aspect, the invention provides a composition that contains zoledronic acid in a solid form, wherein at least 80% by weight of the solid zoledronic acid is its crystalline trihydrate of the free acid of zoledronic acid having an X-ray diffraction pattern with a peak at an angle of refraction 2 θ of 9.2 and 10.3 ± 0.2 as depicted in Figure 7. Various embodiments and variants are provided.

In accordance with yet another aspect, the invention provides a pharmaceutical composition that includes crystalline trihydrate of the free acid of zoledronic acid and a pharmaceutically acceptable carrier or diluent. Preferably, the pharmaceutical composition is for oral administration.

In accordance with one aspect, the invention provides a crystalline anhydrous form of the free acid of zoledronic acid. Preferably, the crystalline anhydrous form of the free acid of zoledronic acid has an X-ray diffraction pattern with a peak at an angle of refraction 2 θ of 10.6, 12.9, 13.2, 16.2, 18.0, 21.2 ± 0.2 as depicted in Figure 7.

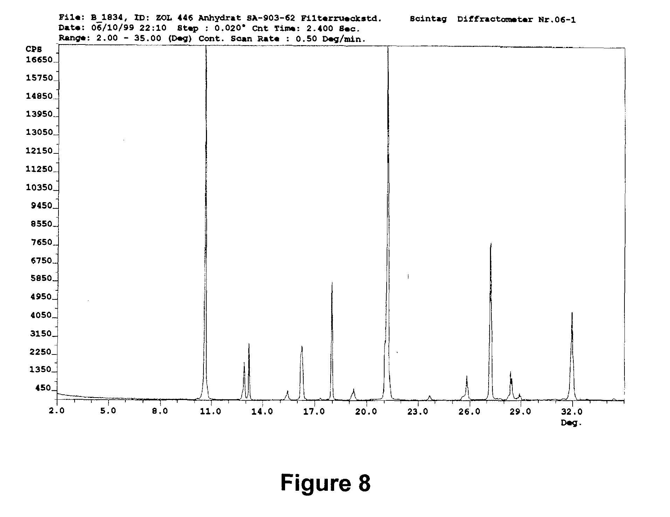

In accordance with yet another aspect, the invention provides a composition that contains zoledronic acid in a solid form, wherein at least 80% by weight of the solid zoledronic acid is its crystalline anhydrous form of the free acid of zoledronic acid having an X-ray diffraction pattern with a peak at an angle of refraction 2 θ of 10.6 ± 0.2 as depicted in Figure 8. Various embodiments and variants are provided.

In accordance with yet another aspect, the invention provides a pharmaceutical composition that includes crystalline anhydrous form of the free acid of zoledronic acid and a pharmaceutically acceptable carrier or diluent. Preferably, the pharmaceutical composition is for oral administration.

In accordance with yet another aspect, the invention also relates to the amorphous form of the free acid of zoledronic acid, the process for preparation of the amorphous form of the free acid of zoledronic acid, compositions containing the amorphous form of the free acid of zoledronic acid, and the use of the amorphous form of the free acid of zoledronic acid in diagnostic methods or therapeutic treatment of warm-blooded animals, especially humans.

### Brief Description of the Drawings

FIG. 1 shows the X-ray powder diffraction diagram of the crystalline form of the 1:2 calcium salt of zoledronic acid.
FIG. 2 shows the X-ray powder diffraction diagram of the crystalline form of the 1:1 calcium salt of zoledronic acid.
FIG. 3 shows the X-ray powder diffraction diagram of the crystalline form I of the 1:2 zinc salt of zoledronic acid.
FIG. 4 shows the X-ray powder diffraction diagram of crystalline form II of the 1:2 zinc salt of zoledronic acid.
FIG. 5 shows the X-ray powder diffraction diagram of crystalline form of the 1:2 magnesium salt of zoledronic acid.
FIG. 6 shows the X-ray powder diffraction diagram of the crystalline form of the monohydrate of the free acid of zoledronic acid.
FIG. 7 shows the X-ray powder diffraction diagram of the crystalline form of the trihydrate of the free acid of zoledronic acid.
FIG. 8 shows the X-ray powder diffraction diagram of the crystalline form of the anhydrous form of the free acid of zoledronic acid.

### Detailed Description of the Invention

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

For the purposes of the present invention, the following terms are defined below.

"Pharmaceutically acceptable" means that which is useful in preparing a pharmaceutical composition that is generally non-toxic and is not biologically undesirable and includes that which is acceptable for veterinary use and/or human pharmaceutical use.

"Anti-solvent" is a solvent which when added to an existing solution of a substance reduced the solubility of the substance.

The term "composition" includes, but is not limited to, a powder, a solution, a suspension, a gel, an ointment, an emulsion and/or mixtures thereof. The term "composition" is intended to encompass a product containing the specified ingredients in the specified amounts, as well as any product, which results, directly or indirectly, from combination of the specified ingredients in the specified amounts. A "composition" may contain a single compound or a mixture of compounds. A "compound" is a chemical substance that includes molecules of the same chemical structure.

The term "pharmaceutical composition" is intended to encompass a product comprising the active ingredient(s), pharmaceutically acceptable excipients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing the active ingredient, additional active ingredient(s) and pharmaceutically acceptable excipients.

The term "excipient" means a component of a pharmaceutical product that is not the active ingredient, such as filler, diluent and carrier. The excipients that are useful in preparing a pharmaceutical composition are preferably generally safe, non-toxic and neither biologically nor otherwise undesirable, and are acceptable for veterinary use, as well as human pharmaceutical use. "A pharmaceutically acceptable excipient", as used in the specification and claims, includes both one and more than one such excipient.

"Therapeutically effective amount" means the amount of a compound that, when administered for treating or preventing a disease, is sufficient to effect such treatment or prevention for the disease. The "therapeutically effective amount" will vary depending on the compound, the disease and its severity and the age, weight, etc., of the patient to be treated.

When referring to a chemical reaction, the terms "treating", "contacting" and "reacting" are used interchangeably herein and refer to adding or mixing two or more reagents under appropriate conditions to produce the indicated and/or desired product. It should be appreciated that the reaction which produces the indicated and/or desired product may not necessarily result directly from the combination of two reagents which were initially added, i.e., there may be one or more intermediates which are produced in the mixture which ultimately leads to the formation of the indicated and/or desired product.

The term "substantially free of" in reference to a composition, as used herein, means that the substance form which the composition is free of cannot be detected by methods known to those skilled in the art.

The term "essentially pure" is understood in the context of the present invention to mean especially that at least 90%, preferably at least 95% by weight of the crystals of an acid addition salt of formula (I) are present in the crystal form according to the invention.

Zoledronic acid is known as 1-hydroxy-2-(imidazol-1-yl)-ethane-1,1-diphosphonic acid and has the following chemical structure:

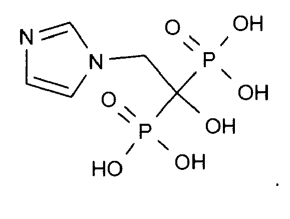

U.S. Patent No. 4,939,130 (the '130 patent) claims zoledronic acid. The invention relates especially to a particular form preferably that which is referred to hereinafter as crystalline form I of the calcium salt of zoledronic acid, crystalline form II of the zinc salt of zoledronic acid, crystalline form III of the magnesium salt of zoledronic acid, and crystalline form IV of the free acid monohydrate of zoledronic acid, described above.

Different solid forms of the same drug may exhibit different properties, including characteristics that have functional implications with respect to their use as drug may have substantial differences in such pharmaceutically important properties as dissolution rates and bioavailability. Likewise, different polymorphs may have different processing properties, such as hydroscopisity, flowability and the like, which could affect their suitability as active pharmaceuticals for commercial production.

X-ray powder diffraction patterns was measured on a Scintag INC X1 with CuK alpha radiation source. The X-ray diffraction pattern depicted in FIG. 1 is summarized in Table 1.

**Table 1. Powder X-Ray Diffraction Peaks for the Crystalline Form of the 1:2 Calcium Salt of Zoledronic Acid**

| **° deg 2 θ** | **d-spacing (Å)** | **Relative intensity** |
|---|---|---|
| 7.8 | 11.35 | high |
| 8.4 | 10.55 | low |
| 9.3 | 9.53 | high |
| 11.5 | 7.73 | high |
| 14.3 | 6.24 | high |
| 17.8 | 5.04 | medium |
| 19.4 | 4.64 | medium |
| 23.1 | 3.93 | medium |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θangle assignments reported herein. The assigned margin of error, in a preferred variant, crystalline form of the 1:2 calcium salt of zoledronic acid is approximately ± 0.2 for each of the peak assignments.

One or more of physical properties and/or spectroscopic properties can be the basis for characterizing the crystal or polymorphic forms of the crystalline form of the 1:2 calcium salt of zoledronic acid.

The invention also provides a composition containing solid crystalline form of the 1:2 calcium salt of zoledronic acid, which is at least 80%, by total weight of the composition. The preferred form of this composition is solid crystalline form of the 1:2 calcium salt of zoledronic acid powder suitable for use as active ingredient in formulating pharmaceutical products. The remainder of the composition, i.e., 20% or less of the total weight of the calcium salt of zoledronic acid may be, e.g., other crystalline forms of low water soluble salts of zoledronic acid. In one specific embodiment, the composition contains at least 90% of the crystalline form of the 1:2 calcium salt of zoledronic acid with respect to the total weight of the composition. In another specific embodiment, the composition contains at least 95% of the crystalline form of the 1:2 calcium salt of zoledronic acid with respect to total weight of the solid in the composition.

X-ray powder diffraction patterns was measured on a STOE STAPI P powder diffraction system with CuK alpha radiation source. The X-ray diffraction pattern depicted in FIG. 2 is summarized in Table 2.

**Table 2. Powder X-Ray Diffraction Peaks for the Crystalline Form of the 1:1 Calcium Salt of Zoledronic Acid**

| **° deg 2 θ** | **d-spacing (Å)** | **Relative intensity** |
|---|---|---|
| 5.7 | 15.56 | low |
| 6.5 | 13.64 | strong |
| 9.0 | 9.79 | strong |
| 10.6 | 8.31 | medium |
| 12.9 | 6.87 | medium |
| 17.4 | 5.10 | medium |
| 18.1 | 4.89 | low |
| 18.8 | 4.72 | low |
| 19.7 | 4.50 | medium |
| 20.2 | 4.39 | medium |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θangle assignments reported herein. The assigned margin of error, in a preferred variant, crystalline form of the 1:1 calcium salt of zoledronic acid is approximately ± 0.2 for each of the peak assignments.

One or more of physical properties and/or spectroscopic properties can be the basis for characterizing the crystal or polymorphic forms of the crystalline form of the 1:1 calcium salt of zoledronic acid.

The invention also provides a composition containing solid crystalline form of the 1:1 calcium salt of zoledronic acid, which is at least 80%, by total weight of the composition. The preferred form of this composition is solid crystalline form of the 1:1 calcium salt of zoledronic acid powder suitable for use as active ingredient in formulating pharmaceutical products. The remainder of the composition, i.e., 20% or less of the total weight of the calcium salt of zoledronic acid may be, e.g., other crystalline forms of low water soluble salts of zoledronic acid. In one specific embodiment, the composition contains at least 90% of the crystalline form of the 1:1 calcium salt of zoledronic acid with respect to the total weight of the composition. In another specific embodiment, the composition contains at least 95% of the crystalline form of the 1:1 calcium salt of zoledronic acid with respect to total weight of the solid in the composition.

X-ray powder diffraction patterns was measured on a Scintag INC X 1 with CuK alpha radiation source. The X-ray diffraction pattern depicted in FIG. 3 is summarized in Table 3.

**Table 3. Powder X-Ray Diffraction Peaks for the Crystalline Form I of the 1:2 Zinc Salt of Zoledronic Acid**

| **° deg 2 θ** | **d-spacing (Å)** | **Relative intensity** |
|---|---|---|
| 9.2 | 9.64 | weak |
| 9.5 | 9.33 | medium |
| 11.7 | 7.60 | strong |
| 15.5 | 5.76 | strong |
| 18.1 | 4.96 | strong |
| 20.5 | 4.40 | medium |
| 23.7 | 3.83 | medium |
| 24.3 | 3.74 | medium |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θ angle assignments reported herein. The assigned margin of error, in a preferred variant, crystalline form I of the 1:2 zinc salt of zoledronic acid is approximately ± 0.2 for each of the peak assignments.

One or more of physical properties and/or spectroscopic properties can be the basis for characterizing the crystal or polymorphic forms of the crystalline form I of the 1:2 zinc salt of zoledronic acid.

The invention also provides a composition containing solid crystalline form I of the 1:2 zinc salt of zoledronic acid, which is at least 80%, by total weight of the composition. The preferred form of this composition is solid crystalline form I of the 1:2 zinc salt of zoledronic acid powder suitable for use as active ingredient in formulating pharmaceutical products. The remainder of the composition, i.e., 20% or less of the total weight of the zinc salt of zoledronic acid may be, e.g., other crystalline forms of low water soluble salts of zoledronic acid. In one specific embodiment, the composition contains at least 90% of the crystalline form I of the 1:2 zinc salt of zoledronic acid with respect to the total weight of the composition. In another specific embodiment, the composition contains at least 95% of the crystalline form I of the 1:2 zinc salt of zoledronic acid with respect to total weight of the solid in the composition.

X-ray powder diffraction patterns was measured on a STOE STAPI P powder diffraction system with CuK alpha radiation source. The X-ray diffraction pattern depicted in FIG. 4 is summarized in Table 4.

**Table 4. Powder X-Ray Diffraction Peaks for the Crystalline Form II of the 1:2 Zinc Salt of Zoledronic Acid**

| **° deg 2 θ** | **d-spacing (Å)** | **Relative intensity** |
|---|---|---|
| 9.1 | 9.70 | strong |
| 13.0 | 6.82 | weak |
| 17.7 | 4.99 | medium |
| 18.0 | 4.90 | weak |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θ angle assignments reported herein. The assigned margin of error, in a preferred variant, crystalline form II of the 1:2 zinc salt of zoledronic acid is approximately ± 0.2 for each of the peak assignments.

One or more of physical properties and/or spectroscopic properties can be the basis for characterizing the crystal or polymorphic forms of the crystalline form II of the 1:2 zinc salt of zoledronic acid.

The invention also provides a composition containing solid crystalline form II of the 1:2 zinc salt of zoledronic acid, which is at least 80%, by total weight of the composition. The preferred form of this composition is solid crystalline form II of the 1:2 zinc salt of zoledronic acid powder suitable for use as active ingredient in formulating pharmaceutical products. The remainder of the composition, i.e., 20% or less of the total weight of the zinc salt of zoledronic acid may be, e.g., other crystalline forms of low water soluble salts of zoledronic acid. In one specific embodiment, the composition contains at least 90% of the crystalline form II of the 1:2 zinc salt of zoledronic acid with respect to the total weight of the composition. In another specific embodiment, the composition contains at least 95% of the crystalline form II of the 1:2 zinc salt of zoledronic acid with respect to total weight of the solid in the composition.

X-ray powder diffraction patterns was measured on a STOE STAPI P powder diffraction system with CuK alpha radiation source. The X-ray diffraction pattern depicted in FIG. 5 is summarized in Table 5.

**Table 5. Powder X-Ray Diffraction Peaks for the Crystalline Form of the 1:2 Magnesium Salt of Zoledronic Acid**

| **° deg 2 θ** | **d-spacing (Å)** | **Relative intensity** |
|---|---|---|
| 4.5 | 19.64 | medium |
| 6.1 | 14.50 | strong |
| 7.7 | 11.50 | strong |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θ angle assignments reported herein. The assigned margin of error, in a preferred variant, crystalline form of the 1:2 magnesium salt of zoledronic acid is approximately ± 0.2 for each of the peak assignments.

One or more of physical properties and/or spectroscopic properties can be the basis for characterizing the crystal or polymorphic forms of the crystalline form of the 1:2 magnesium salt of zoledronic acid.

The invention also provides a composition containing solid crystalline form of the 1:2 magnesium salt of zoledronic acid, which is at least 80%, by total weight of the composition. The preferred form of this composition is solid crystalline form of the 1:2 magnesium salt of zoledronic acid powder suitable for use as active ingredient in formulating pharmaceutical products. The remainder of the composition, i.e., 20% or less of the total weight of the magnesium salt of zoledronic acid may be, e.g., other crystalline forms of low water soluble salts of zoledronic acid. In one specific embodiment, the composition contains at least 90% of the crystalline form of the 1:2 magnesium salt of zoledronic acid with respect to the total weight of the composition. In another specific embodiment, the composition contains at least 95% of the crystalline form of the 1:2 magnesium salt of zoledronic acid with respect to total weight of the solid in the composition.

X-ray powder diffraction patterns was measured on a Scintag INC X1 with CuK alpha radiation source. The X-ray diffraction pattern depicted in FIG. 6 is summarized in Table 6.

**Table 6. Powder X-Ray Diffraction Peaks for the Crystalline Monohydrate of the Free Acid of Zoledronic Acid**

| **° deg 2** θ | **d-spacing (Å)** | **Relative intensity** |
|---|---|---|
| 12.0 | 7.41 | medium |
| 12.8 | 6.95 | strong |
| 15.7 | 5.69 | medium |
| 18.8 | 4.78 | medium |
| 21.2 | 4.26 | medium |
| 21.7 | 4.17 | medium |
| 22.9 | 3.96 | medium |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θ angle assignments reported herein. The assigned margin of error, in a preferred variant, crystalline monohydrate of the free acid of zoledronic acid is approximately ± 0.2 for each of the peak assignments.

One or more of physical properties and/or spectroscopic properties can be the basis for characterizing the crystal or polymorphic forms of the crystalline monohydrate of the free acid of zoledronic acid.

The invention also provides a composition containing solid crystalline monohydrate of the free acid of zoledronic acid, which is at least 80%, by total weight of the composition. The preferred form of this composition is solid crystalline monohydrate of the free acid of zoledronic acid powder suitable for use as active ingredient in formulating pharmaceutical products. The remainder of the composition, i.e., 20% or less of the total weight of the crystalline monohydrate of the free acid of zoledronic acid may be, e.g., other crystalline forms of low water soluble salts of zoledronic acid. In one specific embodiment, the composition contains at least 90% of the crystalline form VI which is the crystalline monohydrate of the free acid of zoledronic acid with respect to the total weight of the composition. In another specific embodiment, the composition contains at least 95% of the crystalline monohydrate of the free acid of zoledronic acid with respect to total weight of the solid in the composition.

X-ray powder diffraction patterns was measured on a Scintag INC X1 with CuK alpha radiation source. The X-ray diffraction pattern depicted in FIG. 7 is summarized in Table 7.

**Table 7. Powder X-Ray Diffraction Peaks for the Crystalline Trihydrate of the Free Acid of Zoledronic Acid**

| **° deg 2 θ** | **d-spacing (Å)** | **Relative intensity** |
|---|---|---|
| 9.2 | 9.64 | weak |
| 10.3 | 8.62 | weak |
| 10.7 | 8.30 | weak |
| 13.3 | 6.70 | weak |
| 16.3 | 5.49 | strong |
| 18.4 | 4.88 | weak |
| 21.5 | 4.20 | weak |
| 21.8 | 4.15 | weak |
| 22.8 | 3.98 | medium |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θ angle assignments reported herein. The assigned margin of error, in a preferred variant, crystalline trihydrate of the free acid of zoledronic acid is approximately ± 0.2 for each of the peak assignments.

One or more of physical properties and/or spectroscopic properties can be the basis for characterizing the crystal or polymorphic forms of the crystalline trihydrate of the free acid of zoledronic acid.

The invention also provides a composition containing solid crystalline trihydrate of the free acid of zoledronic acid, which is at least 80%, by total weight of the composition. The preferred form of this composition is solid crystalline trihydrate of the free acid of zoledronic acid powder suitable for use as active ingredient in formulating pharmaceutical products. The remainder of the composition, i.e., 20% or less of the total weight of the crystalline trihydrate of the free acid of zoledronic acid may be, e.g., other crystalline forms of low water soluble salts of zoledronic acid. In one specific embodiment, the composition contains at least 90% of the crystalline trihydrate of the free acid of zoledronic acid with respect to the total weight of the composition. In another specific embodiment, the composition contains at least 95% of the crystalline trihydrate of the free acid of zoledronic acid with respect to total weight of the solid in the composition.

X-ray powder diffraction patterns was measured on a Scintag INC X1 with CuK alpha radiation source. The X-ray diffraction pattern depicted in FIG. 8 is summarized in Table 8.

**Table 8. Powder X-Ray Diffraction Peaks for the crystalline anhydrous form of the free acid of zoledronic acid**

| **° deg 2 θ** | **d-spacing (Å)** | **Relative intensity (%)** |
|---|---|---|
| 10.6 | 8.38 | strong |
| 12.9 | 6.90 | weak |
| 13.2 | 6.75 | weak |
| 16.2 | 5.52 | weak |
| 18.0 | 4.99 | medium |
| 21.2 | 5.52 | strong |

It should be kept in mind that slight variations in observed 2 θ angles or d-spacing values are expected based on the specific diffractometer employed, the analyst and the sample preparation technique. More variation is expected for the relative peak intensities. Identification of the exact crystalline form of a compound should be based primarily on observed 2 θ angles with lesser importance attributed to relative peak intensities.

Some margin of error is present in each of the 2 θ angle assignments reported herein. The assigned margin of error, in a preferred variant, anhydrous form of the free acid of zoledronic acid is approximately ± 0.2 for each of the peak assignments.

One or more of physical properties and/or spectroscopic properties can be the basis for characterizing the crystal or polymorphic forms of the anhydrous form of the free acid of zoledronic acid.

The invention also provides a composition containing solid anhydrous form of the free acid of zoledronic acid, which is at least 80%, by total weight of the composition. The preferred form of this composition is solid anhydrous form of the free acid of zoledronic acid powder suitable for use as active ingredient in formulating pharmaceutical products. The remainder of the composition, i.e., 20% or less of the total weight of the anhydrous form of the free acid of zoledronic acid may be, e.g., other crystalline forms of low water soluble salts of zoledronic acid. In one specific embodiment, the composition contains at least 90% of the anhydrous form of the free acid of zoledronic acid with respect to the total weight of the composition. In another specific embodiment, the composition contains at least 95% of the anhydrous form of the free acid of zoledronic acid with respect to total weight of the solid in the composition.

The invention also provides a process for making the crystalline form of the 1:2 calcium salt of zoledronic acid, the process including:
(a) providing a solution of zoledronic acid in either a protic or an aprotic solvent;
(b) adding of CaCl₂ in a molar ratio of one calcium to two zoldedronic acid and, then, cooling to form a precipitate; and
(c) isolating the precipitate, which is the crystalline form of the 1:2 calcium salt of zoledronic acid.

The invention also provides a process for making the crystalline form of the 1:1 calcium salt of zoledronic acid, the process including:
(a) providing a solution of zoledronic acid in either a protic or an aprotic solvent;
(b) adding of CaCl₂ in an equimolar ratio of one calcium to one zoldedronic acid and, then, cooling to form a precipitate; and
(c) isolating the precipitate, which is crystalline form of the 1:1 calcium salt of zoledronic acid.

The invention also provides for a process for making the crystalline form I of the 1:2 zinc salt of zoledronic acid, the process including:
(a) providing a solution of zoledronic acid in either a protic or an aprotic solvent;
(b) adding of ZnCl₂ in a molar ratio of one zinc to two zoldedronic acid and, then, cooling to form a precipitate; and
(c) isolating the precipitate, which is crystalline form I of the 1:2 zinc salt of zoledronic acid.

The invention also provides for a process for making the crystalline form II of the 1:2 zinc salt of zoledronic acid, the process including:
(a) providing a solution of zoledronic acid in either a protic or an aprotic solvent;
(b) adding of ZnCl₂ in a molar ratio of one zinc to two zoldedronic acid and, then, cooling to form a precipitate; and
(c) isolating the precipitate, which is crystalline form II of the 1:2 zinc salt of zoledronic acid.

The invention also provides for a process for making the crystalline form of the 1:2 magnesium salt of zoledronic acid, the process including:
(a) providing a solution of zoledronic acid in either a protic or an aprotic solvent;
(b) adding of MgCl₂ in a molar ratio of one magnesium to two zoldedronic acid and, then, cooling to form a precipitate; and
(c) isolating the precipitate, which is crystalline form of the 1:2 magnesium salt of zoledronic acid.

The invention also provides for a process for making the crystalline form of the monohydrate of the free acid of zoledronic acid, the process including:
(a) providing a solution of zoledronic acid in either a protic or an aprotic solvent;
(b) seeding with crystalline form of the monohydrate of the free acid of zoledronic acid and contacting the reaction mixture with an alcohol solvent to form a precipitate; and
(c) isolating the precipitate, which is crystalline form of the monohydrate of the free acid of zoledronic acid.

The invention also provides for a process for making the crystalline form of the trihydrate of the free acid of zoledronic acid, the process including:
(a) providing a suspension of the anhydrous form or the monohydrate of zoledronic acid in either a mixture of a protic or an aprotic solvent with water;
(b) equilibration of the suspension
(c) isolating the precipitate, which is crystalline form of the trihydrate of the free acid of zoledronic acid.

The invention also provides for a process for making the crystalline form of the anhydrous form of the free acid of zoledronic acid, the process including:
(a) providing a solution of zoledronic acid in either a protic or an aprotic solvent;
(b cooling the solution of free acid of zoledronic acid and and contacting the reaction mixture with an anti-solvent to form a precipitate; and
(c) isolating the precipitate, which is crystalline form of the anhydrous form of the free acid of zoledronic acid.

Non-limiting examples of the protic or aprotic solvents are listed in the Table below:

| **Examples** |
|---|
| Acetone |
| Benzyl Alcohol |
| Ethanol |
| Dimethyl Sulfoxide (DMSO) |
| Dimethyl formamide (DMF) |
| THF |
| Acetic acid |
| Polyethylene glycol (PEG 200) |

Also provided are pharmaceutical compositions containing crystalline form of the 1:2 calcium salt of zoledronic acid, the crystalline form of the 1:1 calcium salt of zoledronic acid, the crystalline form I of the 1:2 zinc salt of zoledronic acid, the crystalline form II of the 1:2 zinc salt of zoledronic acid, the crystalline form of the 1:2 magnesium salt of zoledronic acid, the crystalline form of the monohydrate of the free acid of zoledronic acid, the crystalline form of the trihydrate of the free acid of zoledronic acid, the crystalline form of the anhydrous form of the free acid of zoledronic acid which is the anhydrous form of the free acid of zoledronic acid and a pharmaceutically acceptable carrier. In addition to the active compound, the pharmaceutical composition include one or more pharmaceutically acceptable carriers, also known as excipients, which ordinarily lack pharmaceutical activity, but have various useful properties which may, e.g., enhance the stability, sterility, bioavailability and ease of formulation of a pharmaceutical composition. These carriers are pharmaceutically acceptable, meaning that they are not harmful to humans or animals when taken appropriately and are compatible with other ingredients in a given formulation. The carriers may be solid, semi-solid or liquid, and may be formulated with the compound in bulk, but ultimately in the form of a unit-dose formulation, i.e., a physically discrete until containing a specific amount of active ingredient, such as a tablet or capsule. The pharmaceutical compositions may include, in addition to a compound of this invention, one or more active pharmaceutical compounds.

The pharmaceutical compositions may be in the form of suspensions, solutions, elixirs, aerosols or solid dosage forms.

The pharmaceutical compositions are contemplated in various formulations suitable for various modes of administration including, but not limited to, inhalation, oral, rectal, parenteral (including subcutaneous, intradermal, intramuscular and intravenous), implantable and transdermal administration. The most suitable route of administration in an given case depends on the duration of the subject's condition, the length of treatment desired, the nature and severity of the condition being treated, and the particular formulation that is being used. The formulations may be in bulk or in unit dosage form, and may be prepared by methods well-known in the art for a given formulation.

The amount of active ingredient included in a unit dosage form depends on the type of formulation in which the active ingredient is presented. A pharmaceutical composition will generally contain about 0.1 % by weight to about 99% by weight of the active ingredient, preferably about 1% by weight to 50% by weight for oral administration and about 0.2% by weight to about 20% by weight for parenteral administration.

Formulations suitable for oral administration include capsules (hard and soft), cachets, lozenges, syrups, suppositories and tablets, each containing a predetermined amount of the active compound; as a powder or granules, as a solution or a suspension in an aqueous or non-aqueous liquid; or as an oil-in-water or water-in-oil emulsion. Such formulations may be prepared by any suitable method of pharmacy that includes the step of bringing into association the active compound and a suitable carrier or carriers. The amount of active ingredient per unit dosage of solid formulations may be as described in prior art for preparations of zoledronic acid.

In another aspect, the invention also provides methods of treatment using the compounds and the pharmaceutical compositions of this invention. By subject is meant a human or an animal, preferably human. Animals contemplated by this invention include any animal safely treatable by compounds of this invention. Most notably, crystalline form of the 1:2 calcium salt of zoledronic acid, the crystalline form of the 1:1 calcium salt of zoledronic acid, the crystalline form I of the 1:2 zinc salt of zoledronic acid, the crystalline form II of the 1:2 zinc salt of zoledronic acid, the crystalline form of the 1:2 magnesium salt of zoledronic acid, the crystalline form of the monohydrate of the free acid of zoledronic acid, the crystalline form of the trihydrate of the free acid of zoledronic acid, the crystalline form of the anhydrous form of the free acid of zoledronic acid, which may be extremely useful for cancer treatment.

The present invention relates especially to crystalline form of the 1:2 calcium salt of zoledronic acid, the crystalline form of the 1:1 calcium salt of zoledronic acid, the crystalline form I of the 1:2 zinc salt of zoledronic acid, the crystalline form II of the 1:2 zinc salt of zoledronic acid, the crystalline form of the 1:2 magnesium salt of zoledronic acid, the crystalline form of the monohydrate of the free acid of zoledronic acid, the crystalline form of the trihydrate of the free acid of zoledronic acid, the crystalline form of the anhydrous form of the free acid of zoledronic acid disclosed herein for the treatment of one of the said diseases or in the preparation of a pharmacological agent for the treatment thereof.

The invention relates also to a process for the treatment of warm-blooded animals suffering from said diseases, especially a tumor disease, wherein a quantity of the crystalline form of the 1:2 calcium salt of zoledronic acid, the crystalline form of the 1:1 calcium salt of zoledronic acid, the crystalline form I of the 1:2 zinc salt of zoledronic acid, the crystalline form II of the 1:2 zinc salt of zoledronic acid, the crystalline form of the 1:2 magnesium salt of zoledronic acid, the crystalline form of the monohydrate of the free acid of zoledronic acid, the crystalline form of the trihydrate of the free acid of zoledronic acid, the crystalline form of the anhydrous form of the free acid of zoledronic acid, which is effective against the disease concerned, especially a quantity with anti-proliferative and especially tumor-inhibiting efficacy, is administered to warm-blooded animals in need of such treatment. The invention relates moreover to the use of crystalline form of the 1:2 calcium salt of zoledronic acid, the crystalline form of the 1:1 calcium salt of zoledronic acid, the crystalline form I of the 1:2 zinc salt of zoledronic acid, the crystalline form II of the 1:2 zinc salt of zoledronic acid, the crystalline form of the 1:2 magnesium salt of zoledronic acid, the crystalline form of the monohydrate of the free acid of zoledronic acid, the crystalline form of the trihydrate of the free acid of zoledronic acid, the crystalline form of the anhydrous form of the free acid of zoledronic acid for the preparation of pharmaceutical compositions for use in treating the human or animal body, especially for the treatment of a variety of solid tumors and more specifically, e.g., breast cancer, colon cancer, ovarian cancer and leukemia. Depending on species, age, individual condition, mode of administration and the clinical picture in question, effective doses, e.g., daily doses of about 1-2,500 mg, preferably 1-1,000 mg, especially 5-500 mg, are administered to warm-blooded animals of about 70 kg body weight.

The invention relates also to pharmaceutical preparations which contain an effective amount, especially an effective amount for prevention or treatment of one of the said diseases, of crystalline form of the 1:2 calcium salt of zoledronic acid, the crystalline form of the 1:1 calcium salt of zoledronic acid, the crystalline form I of the 1:2 zinc salt of zoledronic acid, the crystalline form II of the 1:2 zinc salt of zoledronic acid, the crystalline form of the 1:2 magnesium salt of zoledronic acid, the crystalline form of the monohydrate of the free acid of zoledronic acid, the crystalline form of the trihydrate of the free acid of zoledronic acid, the crystalline form of the anhydrous form of the free acid of zoledronic acid or a combination of all crystalline forms together with pharmaceutically acceptable carriers which are suitable for topical; enteral, e.g., oral or rectal; or parenteral administration and may be inorganic or organic and solid or liquid. Especially tablets or gelatin capsules containing the active substance together with diluents, e.g., lactose, dextrose, sucrose, mannitol, sorbitol, cellulose and/or glycerin; and/or lubricants, e.g., silica, talc, stearic acid or salts thereof, typically magnesium or calcium stearate; and/or PEG, are, used for oral administration. Tablets may likewise contain binders, e.g., magnesium aluminum silicate, starches, typically corn, wheat or rice starch, gelatin, methylcellulose, sodium carboxymethylcellulose and/or polyvinylpyrrolidone; and, if so desired, disintegrants, e.g., starches, agar, alginic acid or a salt thereof, typically sodium alginate; and/or effervescent mixtures, or adsorbents, coloring agents, flavors and sweetening agents. The pharmacologically active compounds of the present invention may further be used in the form of preparations for parenteral administration or infusion solutions. Such solutions are preferably isotonic aqueous solutions or suspensions, these possibly being prepared before use, e.g., in the case of lyophilised preparations containing the active substance either alone or together with a carrier, e.g., mannitol. The pharmaceutical substances may be sterilised and/or may contain excipients, e.g., preservatives, stabilisers, wetting agents and/or emulsifiers; solubilizers; salts for the regulation of osmotic pressure; and/or buffers. The present pharmaceutical preparations which, if so desired, may contain further pharmacologically active substances, such as antibiotics, are prepared in a manner known *per se*, e.g., by means of conventional mixing, granulating, coating, dissolving or lyophilising processes, and contain from about 1-100%, especially from about 1% to about 20%, of the active substance or substances.

The invention is further defined by reference to the following examples describing in detail the preparation of the compound and the compositions of the present invention, as well as their utility. It will be apparent to those skilled in the art, that many modifications, both to materials, and methods, may be practiced with out departing from the purpose and interest of this invention. The examples that follow are not intended to limit the scope of the invention as defined hereinabove or as claimed below.

### EXAMPLES

### Example 1: Process for Making the Crystalline Form of the 1:2 Calcium Salt of Zoledronic acid

In a 2,500 mL 3-necked flask with mechanical stirrer, 50 g zoledronic acid and 1380 mL water are added. This mixture is heated to about 90°C until everything goes into solution. To this clear solution a solution of 19 g calcium chloride dihydrate in 345 mL water is added. The mixture is cooled to room temperature and stirred over night. The precipitated Ca salt is then isolated by filtration, washing with water and dried in a vacuum oven at 50°C over night. This gives 49.8 g Ca salt of the following composition: C: 18.93; H: 3.82; N: 8.65; P: 20.1; Ca: 6.36; H₂O: 8.48. This corresponds to a trihydrate. m.p. >230°C

### Example 2: Process for Making the Crystalline Form of the 1:1 Calcium Salt of Zoledronic Acid

In a Erlenmeyer flask, 2.9 g zoledronic acid are dissolved in 10 mL sodium hydroxide solution 2 N. To this solution a solution of 2.22 g calcium chloride in 80 mL water is added at room temperature. After stirring the mixture at room temperature for some hours the formed white precipitate is filtrated and washed with water. Drying over night in a vacuum oven gives 3.9 g of a white product with the following composition: C: 15.46; H:4.18; N: 7.18; P: 16.3; Ca: 10.6; H₂O: 18.36.

### Example 3: Process for Making the Crystalline Form I of the 1:2 Zinc Salt of Zoledronic Acid

In a 2,500 mL 3-necked flask with mechanical stirrer, 50 g zoledronic acid, 1,382 mL water and 84.5 mL sodium hydroxide solution 2 N are added with stirring. This gives a clear solution. To this solution, a solution of 23.5 g zinc chloride in 346 mL water is added. The resulting white suspension is stirred over night. The precipitated Zn salt is then isolated by filtration, washing with water and dried in a vacuum oven at 50°C over night. This gives 51 g Zn salt with the following composition: C: 18.76; H: 3.38; N: 8.65; P: 20.2; Zn: 11.0 and H₂O: 3.81, which according to XRPD is a mixture of two modifications.

By stirring 46.3 g of this product in 2 L ethanol:water 1:1 over night, filtration and drying in a vacuum oven at 50°C over night 46.9 g of a single modification is obtained. This product has the following composition: C: 18.01; H: 3.62; N: 8.20; P: 19.1; Zn: 10.5 and H₂O: 8.08.

### Example 4: Process for Making the Crystalline Form of the 1:2 Magnesium Salt of Zoledronic Acid

In a 500 mL three-necked flask, 2.9 g zoledronic acid are dissolved in 80 mL water and 10 mL NaOH 2 N. To this solution a solution of 0.408 g magnesium chloride hexahydrate in 80 mL ethanol is added at room temperature. The product crystallizes slowly during stirring the reaction mixture at room temperature. After stirring for some hours, the formed white precipitate is filtrated and washed with ethanol. Drying over night in a vacuum oven gives 2.79 g of a white product with the following composition: C: 17.87; H: 3.22; N: 8.22; P: 18.6; Mg: 1.45; H₂O: 7.73.

### Example 5: Process for Making the Crystalline Form of the Monohydrate of the Free Acid of Zoledronic Acid

About 300 mg of the anhydrous form of zoledronic acid is suspended in about 1 mL of 96% ethanol. The suspension is equilibrated for about 2 hours at about 60°C. The solid precipitate is then isolated by filtration.

### Example 6: Process for Making the Crystalline Form of the Trihydrate of the Free Acid of Zoledronic Acid

About 500 mg of the monohydrate of zoledronic acid is suspended in about 50 mL of water. The suspension is equilibrated over night at room temperature. The solid precipitate is then isolated by filtration.

### Example 7: Process for Making the Crystalline Form of the Anhydrous Form of the Free Acid of Zoledronic Acid

About 322 mg of the monohydrate or of the trihydrate of zoledronic acid are suspended in about 3,300 mL of water and heated until all solids are dissolved. The solution is then cooled to about 55°C and about 1,400 mL of acetone is added. The reaction mixture is further cooled to about room temperature over night under stirring. After further cooling and stirring for about 1 hours at about 0°C the solid precipitate is then isolated by filtration.

## Claims

1. A compound which is a crystalline form of the 1:2 calcium salt of zoledronic acid.

2. The compound according to Claim 1, which shows on X-ray diffraction a peak at an angle of refraction 2 theta (θ), of 7.8 or 9.3 ± 0.2 degrees.

3. The compound according to Claim 1, having an X-ray diffraction pattern, expressed in terms of 2 θ angles, that includes four or more peaks selected from the group consisting of about 7.8, 8.4, 9.3, 11.5,14.3, 17.8, 19.4, 23.1 ± 0.2 degrees.

4. The compound according to Claim 1, having substantially the same X-ray diffraction pattern as shown in Figure 1.

5. The compound according to Claim 1, which remains dry at 95% relative humidity and 25°C.

6. A composition comprising the calcium salt of zoledronic acid as a solid, wherein at least 80% by weight of said solid is crystalline form of the 1:2 calcium salt of zoledronic acid.

7. The composition according to Claim 6, wherein at least 90% by weight of said solid is crystalline form of the 1:2 calcium salt of zoledronic acid.

8. The composition according to Claim 6, wherein at least 95% by weight of said solid is crystalline form of the 1:2 calcium salt of zoledronic acid.

9. A pharmaceutical composition comprising:
(a) the compound of Claim 1; and
(b) a pharmaceutically acceptable carrier or diluent.

10. The pharmaceutical composition according to Claim 9, further comprising one or more pharmaceutically acceptable excipients.

11. The pharmaceutical composition according to Claim 9, which is a dosage form suitable for oral administration.

12. The pharmaceutical composition according to Claim 11, wherein said dosage form is selected from a tablet, capsule or solution.

13. Use of crystalline form of the 1:2 calcium salt of zoledronic acid according to any one of the Claims 1-7, for the preparation of a pharmacological agent for the treatment of a tumor disease.

14. A compound which is a crystalline form of the 1:1 calcium salt of zoledronic acid.

15. The compound according to Claim 14, which shows on X-ray diffraction a peak at an angle of refraction 2 θ, of 6.5 or 9.0 ± 0.2 degrees.

16. The compound according to Claim 14, having an x-ray diffraction pattern, expressed in terms of 2 θ angles, that includes five or more peaks selected from the group consisting of about 7.8, 8.4, 9.3, 11.5, 14.3, 17.8, 19.4, 23.1 ± 0.2 degrees.

17. The compound according to Claim 14, having substantially the same X-ray diffraction pattern as shown in Figure 2.

18. The compound according to Claim 14, which remains dry at 95% relative humidity and 25°C.

19. A composition comprising the zinc salt of zoledronic acid as a solid, wherein at least 80% by weight of said solid is crystalline form of the 1:1 calcium salt of zoledronic acid.

20. The composition according to Claim 19, wherein at least 90% by weight of said solid is crystalline form of the 1:1 calcium salt of zoledronic acid.

21. The composition according to Claim 19, wherein at least 95% by weight of said solid is crystalline form of the 1:1 calcium salt of zoledronic acid.

22. A pharmaceutical composition comprising:
(a) the compound of Claim 15; and
(b) a pharmaceutically acceptable carrier or diluent.

23. The pharmaceutical composition according to Claim 22, further comprising one or more pharmaceutically acceptable excipients.

24. The pharmaceutical composition according to Claim 22, which is a dosage form suitable for oral administration.

25. The pharmaceutical composition according to Claim 24, wherein said dosage form is selected from a tablet, capsule or solution.

26. Use of crystalline form of the 1:1 calcium salt of zoledronic acid according to any one of the Claims 15-22, for the preparation of a pharmacological agent for the treatment of a tumor disease.

27. A compound which is a crystalline form I of the 1:2 zinc salt of zoledronic acid.

28. The compound according to Claim 27, which shows on X-ray diffraction a peak at an angle of refraction 2 θ, of 11.7, 15.5 or 18.1 ± 0.2 degrees.

29. The compound according to Claim 27, having an x-ray diffraction pattern, expressed in terms of 2 θ angles, that includes five or more peaks selected from the group consisting of about 9.2, 9.5, 11.7, 15.5, 18.1, 20.5, 23.7 or 24.3 ± 0.2 degrees.

30. The compound according to Claim 27, having substantially the same X-ray diffraction pattern as shown in Figure 3.

31. The compound according to Claim 27, which remains dry at 95% relative humidity and 25°C.

32. A composition comprising the zinc salt of zoledronic acid as a solid, wherein at least 80% by weight of said solid is crystalline form I of the 1:2 zinc salt of zoledronic acid.

33. The composition according to Claim 32, wherein at least 90% by weight of said solid is crystalline form I of the 1:2 zinc salt of zoledronic acid.

34. The composition according to Claim 32, wherein at least 95% by weight of said solid is crystalline form I of the 1:2 zinc salt of zoledronic acid.

35. A pharmaceutical composition comprising:
(a) the compound of Claim 27; and
(b) a pharmaceutically acceptable carrier or diluent.

36. The pharmaceutical composition according to Claim 35, further comprising one or more pharmaceutically acceptable excipients.

37. The pharmaceutical composition according to Claim 35, which is a dosage form suitable for oral administration.

38. The pharmaceutical composition according to Claim 35, wherein said dosage form is selected from a tablet, capsule or solution.

39. Use of crystalline form I of the 1:2 zinc salt of zoledronic acid according to any one of the Claims 27-32 for the preparation of a pharmacological agent for the treatment of a tumor disease.

40. A compound which is a crystalline form II of the 1:2 zinc salt of zoledronic acid.

41. The compound according to Claim 40, which shows on X-ray diffraction a peak at an angle of refraction 2 θ of 9.1 ± 0.2 degrees.

42. The compound according to Claim 41, having an x-ray diffraction pattern, expressed in terms of 2 θ angles, that includes two or more peaks selected from the group consisting of about 9.1, 13.0, 17.7 or 18.0 ± 0.2 degrees.

43. The compound according to Claim 42, having substantially the same X-ray diffraction pattern as shown in Figure 4.

44. The compound according to Claim 40, which remains dry at 95% relative humidity and 25°C.

45. A composition comprising the crystalline form II of the 1:2 zinc salt of zoledronic acid as a solid, wherein at least 80% by weight of said solid is crystalline form II of the 1:2 zinc salt of zoledronic acid.

46. The composition according to Claim 45, wherein at least 90% by weight of said solid is crystalline form II of the 1:2 zinc salt of zoledronic acid.

47. The composition according to Claim 45, wherein at least 95% by weight of said solid is crystalline form II of the 1:2 zinc salt of zoledronic acid.

48. A pharmaceutical composition comprising:
(a) the compound of Claim 40; and
(b) a pharmaceutically acceptable carrier or diluent.

49. The pharmaceutical composition according to Claim 45, further comprising one or more pharmaceutically acceptable excipients.

50. The pharmaceutical composition according to Claim 45, which is a dosage form suitable for oral administration.

51. The pharmaceutical composition according to Claim 50, wherein said dosage form is selected from a tablet, capsule or solution.

52. Use of crystalline form II of the 1:2 zinc salt of zoledronic acid according to any one of the Claims 40-51, for the preparation of a pharmacological agent for the treatment of a tumor disease.

53. A compound which is a crystalline form of the 1:2 magnesium salt of zoledronic acid.

54. The compound according to Claim 53, having an X-ray diffraction pattern, expressed in terms of 2 θ angles, that includes one or more peaks selected from the group consisting of about 4.5, 6.1 and 7.7 ± 0.2 degrees.

55. The compound according to Claim 53, having substantially the same X-ray diffraction pattern as shown in Figure 5.

56. The compound according to Claim 53, which remains dry at 95% relative humidity and 25°C.

57. A composition comprising the crystalline form of the 1:2 magnesium salt of zoledronic acid as a solid, wherein at least 80% by weight of said solid is crystalline form of the 1:2 magnesium salt of zoledronic acid.

58. The composition according to Claim 57, wherein at least 90% by weight of said solid is crystalline form of the 1:2 magnesium salt of zoledronic acid.

59. The composition according to Claim 57, wherein at least 95% by weight of said solid is crystalline form of the 1:2 magnesium salt of zoledronic acid.

60. A pharmaceutical composition comprising:
(a) the compound of Claim 53; and
(b) a pharmaceutically acceptable carrier or diluent.

61. The pharmaceutical composition according to Claim 60, further comprising one or more pharmaceutically acceptable excipients.

62. The pharmaceutical composition according to Claim 60, which is a dosage form suitable for oral administration.

63. The pharmaceutical composition according to Claim 62, wherein said dosage form is selected from a tablet, capsule or solution.

64. Use of crystalline form of the 1:2 magnesium salt of zoledronic acid according to any one of the Claims 53-60, for the preparation of a pharmacological agent for the treatment of a tumor disease.

65. A compound which is a crystalline form of the monohydrate of the free acid of zoledronic acid.

66. The compound according to Claim 65, which shows on X-ray diffraction a peak at an angle of refraction 2 θ of 12.8 ± 0.2 degrees.

67. The compound according to Claim 65, having an x-ray diffraction pattern, expressed in terms of 2 θ angles, that includes five or more peaks selected from the group consisting of about 12.0, 12.8, 15.7, 18.8, 21.2, 21.7 or 22.9 ± 0.2 degrees.

68. The compound according to Claim 65, having substantially the same X-ray diffraction pattern as shown in Figure 6.

69. The compound according to Claim 65, which remains dry at 95% relative humidity and 25°C.

70. A composition comprising the crystalline form of the monohydrate of the free acid of zoledronic acid as a solid, wherein at least 80% by weight of said solid is crystalline form of the monohydrate of the free acid of zoledronic acid.

71. The composition according to Claim 70, wherein at least 90% by weight of said solid is crystalline form of the monohydrate of the free acid of zoledronic acid.

72. The composition according to Claim 70, wherein at least 95% by weight of said solid is crystalline form of the monohydrate of the free acid of zoledronic acid.

73. A pharmaceutical composition comprising:
(a) the compound of Claim 65; and
(b) a pharmaceutically acceptable carrier or diluent.

74. The pharmaceutical composition according to Claim 73, further comprising one or more pharmaceutically acceptable excipients.

75. The pharmaceutical composition according to Claim 73, which is a dosage form suitable for oral administration.

76. The pharmaceutical composition according to Claim 73, wherein said dosage form is selected from a tablet, capsule or solution.

77. Use of crystalline form of the monohydrate of the free acid of zoledronic acid according to any one of the Claims 65-76, for the preparation of a pharmacological agent for the treatment of a tumor disease.

78. A compound which is a crystalline form of the crystalline form of the trihydrate of the free acid of zoledronic acid.

79. The compound according to Claim 78, which shows on X-ray diffraction a peak at an angle of refraction 2 θ of 16.3 ± 0.2 degrees.

80. The compound according to Claim 78, having an x-ray diffraction pattern, expressed in terms of 2 θ angles, that includes five or more peaks selected from the group consisting of about 9.2, 10.3, 10.7, 13.3, 16.3, 18.4, 21.5, 21.8, 22.8 ± 0.2 degrees.

81. The compound according to Claim 78, having substantially the same X-ray diffraction pattern as shown in Figure 7.

82. The compound according to Claim 78, which remains dry at 95% relative humidity and 25°C.

83. A composition comprising the crystalline form of the trihydrate of the free acid of zoledronic acid as a solid, wherein at least 80% by weight of said solid is crystalline form of the trihydrate of the free acid of zoledronic acid.

84. The composition according to Claim 83, wherein at least 90% by weight of said solid is crystalline form of the trihydrate of the free acid of zoledronic acid.

85. The composition according to Claim 83, wherein at least 95% by weight of said solid is crystalline form of the trihydrate of the free acid of zoledronic acid.

86. A pharmaceutical composition comprising:
(a) the compound of Claim 78; and
(b) a pharmaceutically acceptable carrier or diluent.

87. The pharmaceutical composition according to Claim 86, further comprising one or more pharmaceutically acceptable excipients.

88. The pharmaceutical composition according to Claim 86, which is a dosage form suitable for oral administration.

89. The pharmaceutical composition according to Claim 86, wherein said dosage form is selected from a tablet, capsule or solution.

90. Use of crystalline form of the trihydrate of the free acid of zoledronic acid according to any one of the Claims 79-89, for the preparation of a pharmacological agent for the treatment of a tumor disease.

91. A compound which is a crystalline anhydrous form of the free acid of zoledronic acid.

92. The compound according to Claim 91, which shows on X-ray diffraction a peak at an angle of refraction 2 θ of 10.6 or 21.2 ± 0.2 degrees.

93. The compound according to Claim 91, having an X-ray diffraction pattern, expressed in terms of 2 θ angles, that includes five or more peaks selected from the group consisting of about 10.6, 12.9, 13.2, 16.2, 18.0, 21.2 ± 0.2 degrees.

94. The compound according to Claim 91, having substantially the same X-ray diffraction pattern as shown in Figure 8.

95. The compound according to Claim 91, which remains dry at 95% relative humidity and 25°C.

96. A composition comprising the crystalline form of the anhydrous form of the free acid of zoledronic acid as a solid, wherein at least 80% by weight of said solid is crystalline form of the anhydrous form of the free acid of zoledronic acid.

97. The composition according to Claim 96, wherein at least 90% by weight of said solid is crystalline form of the anhydrous form of the free acid of zoledronic acid.

98. The composition according to Claim 96, wherein at least 95% by weight of said solid is crystalline form of the anhydrous form of the free acid of zoledronic acid.

99. A pharmaceutical composition comprising:
(a) the compound of Claim 91; and
(b) a pharmaceutically acceptable carrier or diluent.

100. The pharmaceutical composition according to Claim 99, further comprising one or more pharmaceutically acceptable excipients.

101. The pharmaceutical composition according to Claim 99, which is a dosage form suitable for oral administration.

102. The pharmaceutical composition according to Claim 99, wherein said dosage form is selected from a tablet, capsule or solution.

103. Use of crystalline form of the anhydrous form of the free acid of zoledronic acid according to any one of the Claims 91-102, for the preparation of a pharmacological agent for the treatment of a tumor disease.
